# EUROPÄISCHE PATENTSCHRIFT

(11) **EP 2 931 705 B1**
(45) Veröffentlichungstag und Bekanntmachung des Hinweises auf die Patenterteilung: **23.03.2016**
(21) Anmeldenummer: 13802625.7
(22) Anmeldetag: 10.12.2013
(51) Int. Cl.: C07C 319/20, C07C 323/62, C07C 323/54

(54) **VERFAHREN ZUR HERSTELLUNG VON 4-HALOGENALKYL-3-MERCAPTO-SUBSTITUIERTEN 2-HYDROXYBENZOESÄURE-DERIVATEN**
METHOD FOR THE PREPARATION OF 4-HALOGENALKYL-3-MERCAPTO-SUBSTITUTED 2-HYDROXY BENZOIC ACID DERIVATIVES
PROCÉDÉ DE FABRICATION DE DÉRIVÉS D'ACIDE 2-HYDROXYBENZOÏQUE 4-HALOGÈNE-ALKYL-3-MERCAPTO-SUBSTITUÉS

(30) Priorität: 14.12.2012 EP 12197102
(43) Veröffentlichungstag der Anmeldung: 21.10.2015
(73) Patentinhaber: Bayer CropScience AG, 40789 Monheim am Rhein (DE)
(72) Erfinder: FORD, Mark James, 61389 Schmitten (DE); KARIG, Gunter, 65926 Frankfurt am Main-Höchst (DE)
(74) Vertreter: BIP Patents
(86) Internationale Anmeldenummer: PCT/EP2013/076014
(87) Internationale Veröffentlichungsnummer: WO 2014/090766

(56) Entgegenhaltungen:
- US-A1- 2011 045 980
- US-A1- 2011 053 779
- M.A. RASHID, ET AL.: "Regioselective synthesis of diaryl sulfides by [3+3] cyclisations of 1,3-bis(trimethylsilyloxy)-1,3-dienes", TETRAHEDRON LETTERS, Bd. 48, Nr. 13, 1. Februar 2007 (2007-02-01), Seiten 2321-2323, XP005908501, Elsevier Science Publishers, Amsterdam, NL ISSN: 0040-4039, DOI: 10.1016/j.tetlet.2007.01.150

## Beschreibung

Die Erfindung betrifft Verfahren zur Herstellung von 4-halogenalkyl-3-mercapto-substituierten 2-Hydroxybenzoesäure-Derivaten sowie deren Verwendung als Zwischenprodukte für die Synthese von Feinchemikalien und von agrochemischen Wirkstoffen.

4-Halogenalkyl-3-mercapto-substituierte 2-Hydroxybenzoesäure-Derivate der Formel (I) stellen ein wichtiges Strukturelement bei einer Vielzahl von agronomisch wirksamen Substanzen dar, wie sie z.B in US 2011/0045980 A1 und US 2011/0053779 A1 offenbart werden.

Tetrahedron Letters 48,(2007),2321-2323 bescreibt die Herstellung ähnlicher Verbindungen ohne Halogen-Substituenten an der Position 4 durch (3+3) Cyclisierung des bestimmten 1,3-Bis(trimethylsilyloxy)-1,3-dienen.

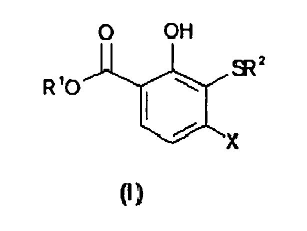

Die in diesen Schriften genannten Verfahren zur Herstellung von 4-halogenalkyl-3-mercapto-substituierten 2-Hydroxybenzoesäure-Derivaten sind jedoch auf eine Durchführung im Labormaßstab beschränkt, da sie eine Reihe von Nachteilen aufweisen und somit nicht für eine industrielle Produktion anwendbar sind: Teure Ausgangsstoffe, Vielzahl von Reaktionsschritten, Reaktionen bei sehr tiefen Temperaturen.

Aufgabe der vorliegenden Erfindung ist die Bereitstellung eines Verfahrens zur Herstellung von 4-halogenalkyl-3-mercapto-substituierten 2-Hydroxybenzoesäure-Derivaten, welches die Nachteile der aus dem Stand der Technik bekannten Verfahren überwindet.

Es wurde nun gefunden, dass 4-halogenalkyl-3-mercapto-substituierte 2-Hydroxybenzoesäure-Derivate sich kostengünstig und in hohen Ausbeuten herstellen lassen durch Reaktion von 4-thiosubstiuierten β-Ketoestern mit Alkoxyvinylhaloalkylketonen bei einer Temperatur von ≥ -30°C in Gegenwart einer Base und eines Lösungsmittels.

Ein Gegenstand vorliegender Erfindung ist somit ein Verfahren zur Herstellung von 4-halogenalkyl-3-mercapto-substituierten 2-Hydroxybenzoesäure-Derivaten der allgemeinen Formel (I), dadurch gekennzeichnet, dass 4-thiosubstiuierte β-Ketoester der Formel (II) mit Alkoxyvinylhaloalkylketonen der Formel (III) bei einer Temperatur von ≥ -30°C in Gegenwart eines tertiären Amins und eines Lösungsmittels umgesetzt werden, und worin die Reste, Symbole und Indices wie nachfolgend definiert sind: 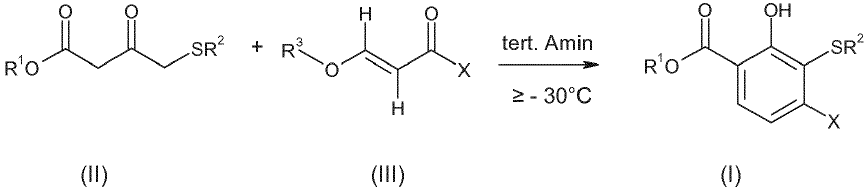
R¹, R² und R³ bedeuten unabhängig voneinander jeweils
durch p Reste aus der Gruppe bestehend aus Fluor, Chlor, (C₁-C₄)-Alkoxy und (C₃-C₇)-Cycloalkyl substituiertes (C₁-C₆)-Alkyl,
durch p Reste aus der Gruppe bestehend aus Fluor, Chlor, (C₁-C₄)-Alkyl, (C₁-C₄)-Alkoxy und (C₃-C₇)-Cycloalkyl substituiertes(C₃-C₇)-Cycloalkyl,
durch p Reste aus der Gruppe bestehend aus Fluor, Chlor, Brom, lod, (C₁-C₄)-Alkyl, (C₁-C₄)-Alkoxy, (C₃-C₇)-Cycloalkyl und (C₁-C₄)-Alkylthio substituiertes Phenyl, 1-Naphthyl, 2-Naphthyl oder 5- oder 6-gliedriges Heteroaryl, wobei dieses Heteroaryl ein oder zwei Heteroatome aus der Gruppe bestehend aus Sauerstoff und Stickstoff enthält,
   - X: bedeutet Halogen-(C₁-C₄)-alkyl,
   - p: bedeutet 0, 1, 2, 3 oder 4.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. (C₃-C₇)-Cycloalkyl steht für Cyclopropyl, Cyclobutyl, Cyclopentyl, Cyclohexyl und Cycloheptyl.

Halogen bedeutet Fluor, Chlor Brom oder lod, bevorzugt Fluor oder Chlor.

Heteroaryl steht beispielsweise für Furanyl, Imidazolyl, Isoxazolyl, Isothiazolyl, Oxazolyl, Pyrazinyl, Pyrimidinyl, Pyridazinyl, Pyridinyl, Pyrrolyl und Pyrazolyl.

Ist eine Gruppe mehrfach durch Reste substituiert, so ist darunter zu verstehen, daß diese Gruppe durch ein oder mehrere gleiche oder verschiedene der genannten Reste substituiert ist.

In dem erfindungsgemäßen Verfahren haben die Reste, Symbole und Indices vorzugsweise folgende Bedeutung:
R¹, R² und R³ bedeuten unabhängig voneinander jeweils
durch p Reste aus der Gruppe bestehend aus Fluor, Chlor, (C₁-C₂)-Alkoxy und (C₃-C₇)-Cycloalkyl substituiertes (C₁-C₄)-Alkyl,
durch p Reste aus der Gruppe bestehend aus Fluor, Chlor, (C₁-C₂)-Alkyl und (C₁-C₂)-Alkoxy substituiertes(C₃-C₇)-Cycloalkyl,
durch p Reste aus der Gruppe bestehend aus Fluor, Chlor, Brom, lod, (C₁-C₂)-Alkyl, (C₁-C₂)-Alkoxy, (C₃-C₇)-Cycloalkyl und (C₁-C₂)-Alkylthio substituiertes Phenyl,
   - X: bedeutet Fluor-(C₁-C₃)-alkyl oder Chlor-(C₁-C₃)-alkyl,
   - p: bedeutet 0, 1, 2, 3 oder 4.

In einer besonders bevorzugten Ausführungsform steht X für CF₃, CF₂H, CFH₂, CFClH, CF₂CH₃, CF(CH₃)₂, CF₂CF₃ oder CH₂CF₃.

Die Verbindungen der Formeln (II) und (III) werden üblicherweise äquimolar eingesetzt. Als tertiäres Amin eignen sich zum Beispiel Trialkylamine wie Triethylamin, Tributylamin, Diisopropylethylamin, Diethylbenzylamin oder Dimethylbenzylamin; substituierte oder unsubstituierte N-Alkylpiperidine, N-Alkylmorpholine oder N-Alkylpyrrolidine. Das tertiäre Amin wird entweder als Einzelverbindung oder als Mischung aus mehreren Aminen katalytisch, äquimolar oder im Überschuss (0.05 bis 5 Äquivalente, bevorzugt 0,1 bis 3 Äquivalente, besonders bevorzugt 0,5 bis 1,5 Äquivalente) bezogen auf die Verbindungen der Formeln (II) und (III) eingesetzt.

Als Lösungsmittel eignen sich aprotische Lösungsmittel wie Ether (bevorzugt Tetrahydrofuran), Nitrile (bevorzugt Acetonitril und Butyronitril), Halogenalkane (bevorzugt Dichlormethan), Ester (bevorzugt Ethylacetat, Butylacetat und Isopropylacetat) und Aromaten (bevorzugt Toluol, Xylol, Chlorbenzol, Benzonitril, Anisol und Benzotrifluorid) und Mischungen dieser genannten Lösungsmittel. Besonders bevorzugt ist Acetonitril.

Die Herstellung der 4-thiosubstiuierten β-Ketoester der Formel (II) ist zum Beispiel in WO 2010/59241 A1 und US 4,521,613 beschrieben.

Die Herstellung der Alkoxyvinylhaloalkylketone der Formel (III) ist beispielsweise in US 2008/269059 A1 beschrieben.

Das erfindungsgemäße Verfahren wird in der Regel so durchgeführt, dass die Verbindungen der Formeln (II) und (III) in einem organischen Lösungsmittel vorgelegt werden und das Amin unter Kühlung zugetropft wird. Das erfindungsgemäße Verfahren kann auch so durchgeführt werden, dass die Verbindung der Formel (II) und das Amin in einem organischen Lösungsmittel vorgelegt werden und die Verbindung der Formel (III) unter Kühlung zugetropft wird. Die letztgenannte Variante ist bevorzugt.

Üblicherweise wird das erfindungsgemäße Verfahren zwischen -30°C und 50°C, bevorzugt im Bereich von -10°C bis 30°C durchgeführt.

Das erfindungsgemäße Verfahren wird üblicherweise drucklos oder unter Druck bis 2 bar durchgeführt. Bevorzugt wird es drucklos durchgeführt.

Die Verbindungen der Formel (II) können auch in Form von Salzen, wie Natrium- oder Kaliumsalzen im erfindungsgemäßen Verfahren eingesetzt werden.

Üblicherweise läßt man nach Zugabe aller Reaktionspartner noch bis zu 96 Stunden, bevorzugt 0,05 bis 24 Stunden, nachrühren.

Es kann vorteilhaft sein, die Reaktionsmischung nach Beendigung der Reaktion zur Aufreinigung mit wässriger Base z.B. Natronlauge oder Kalilauge zu behandeln.

Die Aufarbeitung und Isolierung der Verbindung der Formel (I) erfolgt dann auf üblichen und dem Fachkann bekannten Wegen.

Im Rahmen der vorliegenden Erfindung wurde gefunden, dass die Verbindungen der Formeln (II) und (III) zunächst zu Additionsprodukten der Formel (Ia) reagieren, die mit ihren Tautomeren in einem Gleichgewicht vorliegen und dann zu den Verbindungen der Formel (I) weiterreagieren.

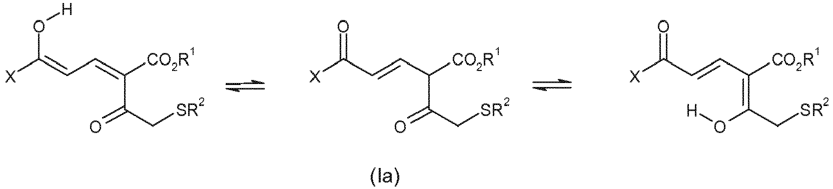

Die Additionsprodukte der Formel (la) sind neu und ebenfalls ein Gegenstand vorliegender Erfindung.

Sofern die Verbindungen der Formel (II) in Form ihrer Salze eingesetzt werden, liegen demzufolge auch die Verbindungen der Formel (la) in Form ihrer Salze vor.
Die nachfolgenden Beispiele erläutern das erfindungsgemäße Verfahren näher.
1. Herstellung von 4-Trifluormethyl-3-methylthio-2-hydroxybenzoesäure-ethylester 100 g Ethyl-4-(methylsulfanyl)-3-oxobutanoat und 27,8 g Triethylamin wurden in 260 ml Toluol vorgelegt und unter Stickstoff auf 0°C gekühlt. Dann wurden 102,8 g 4-Ethoxy-1,1,1-trifluorbut-3-en-2-on inerhalb von 30 Minuten bei 0-7°C zugetropft und 5h weitergerührt. Nach 16 Stunden wurde die Reaktion auf 18°C erwärmt. Die Reaktionsmischung wurde aufkonzentriert, mit 150 ml Acetonitril versetzt und bei 5 mbar eingeengt. Das Rohprodukt wurde unter Rühren und Eiswasserkühlung mit 2 Äquivalenten 10%iger Natronlauge versetzt, 1 h gerührt, der Feststoff abgesaugt, dreimal mit 50ml 3,5%iger Natronlauge gewaschen und trockengesaugt. Der Feststoff wurde dann mit 150 ml Wasser und 150 ml Toluol versetzt, mit ca. 150 ml 10%iger HCl auf pH 1 gestellt, und beim Nachrühren HCl nachdosiert, bis der pH Wert unter 3 bleibt. Dann wurden die filtrierten Phasen getrennt und die Wasserphase nochmal mit Toluol extrahiert. Die kombinierte Toluol-Phasen wurden getrocknet und eingeengt: Ausbeute 122,3 g (78% der Theorie). ¹H-NMR (CDCl₃, 400 MHz): δ = 11.77 ppm (1H, s), 7.93 ppm (1 H, d), 7.23 ppm (1 H, d), 4.47 ppm (2H, q), 2.44 ppm (3H, s), 1.45 ppm (3H, t).
2. Herstellung von 4-Difluormethyl-3-methylthio-2-hydroxybenzoesäure-ethylester 60 g Ethyl-4-(methylsulfanyl)-3-oxobutanoat und 61,3 g 4-Ethoxy-1,1,1-trifluorbut-3-en-2-on wurden in 312 g Acetonitril vorgelegt und unter Stickstoff auf 0°C abgekühlt. Innerhalb von 30 Minuten wurden bei 0°C 17,2 g Triethylamin zugetropft und anschliessend bei 0°C 2 Stunden nachgerührt. Die Reaktion wurde über Nacht bei 20°C gerührt, aufkonzentriert und mit 265 g 10%iger Natronlauge versetzt, 1 h gerührt, der Feststoff abgesaugt, 3 mal mit 50 ml 3,5%iger NaOH gewaschen und trockengesaugt. Der Feststoff wurde dann mit 250 ml Wasser und 250 ml Toluol versetzt, mit 10%iger HCl auf pH 1 gestellt, und beim Nachrühren HCl nachdosiert, bis der pH Wert unter 3 bleibt. Zum Schluss wurden die filtrierten Phasen getrennt und die Wasserphase nochmal mit Toluol extrahiert. Die kombinierte Toluol-Phasen wurden getrocknet und eingeengt: Ausbeute 53,7g (60,1% der Theorie). ¹H-NMR (CDCl₃, 400 MHz): δ = 11.73 ppm (1 H, s), 7.95 ppm (1 H, d), 7.22 ppm (1 H, d), 7.11-7.25-7.39 ppm (1 H, t, CHF2), 4.46 ppm (2H, q), 2.41 ppm (3H, s), 1.44 ppm (3H, t).
3. Herstellung von 4-(Chlor-difluoromethyl)-3-methylthio-2-hydroxybenzoesäureethylester 1,1 g Ethyl-4-(methylsulfanyl)-3-oxobutanoat und 0,294 g Triethylamin in 3,3 ml Toluol unter Argon wurde vorgelegt und auf 0°C abgekühlt. Innerhalb von 15 Minuten bei 0-7°C wurden 1,385 g 4-Ethoxy-1,1,1-trifluorbut-3-en-2-on zugetropft. Die Reaktion wurde auf 20°C erwärmt und über Nacht gerührt. Das Lösungsmittel wurde aufkonzentriert und den Rückstand unter Eiskühlung mit NaOH 4,64g (10%ig) versetzt und 6h gerührt. Der Niederschlag wurde über eine Glassfritte POR3 abgesaugt, mit wenig Eiswasser und anschliessend mit n-Heptan gewaschen. Zu dem noch feuchten Feststoff wurde 10%ige HCl gegeben, mit Toluol überschichtet und gerührt (2h) bis zwei klare Schichten entstanden sind. Die Phasen wurden getrennt und die Wasserphase 1 x mit Toluol extrahiert. Die vereingten organischen Phasen wurden getrocknet und aufkonzentriert. Ausbeute 1,25 g (70,5% der Theorie). ¹H-NMR (CDCl₃, 400 MHz): δ = 11.79 (1 H, s), 7.91 (1 H, d), 7.20 (d, 1 H), 4.47 (2 H, q), 2.45 (3H, s), 1.44ppm(3H,t).
4. Herstellung von 4-(Chlor-difluoromethyl)-3-(4-fluorphenylthio)-2-hydroxybenzoesäureethylester
   In Analogie zu obigen Beispiel 3 wurde 4-(Chlor-difluormethyl)-3-(4-fluorphenylthio)-2-hydroxybenzoesäure-ethylester in 57.6% Ausbeute (der Theorie) hergestellt. ¹H-NMR (CDCl₃, 400 MHz): δ = 11.64 ppm (1 H, s), 8.01 ppm (1 H, d), 7.32 ppm (1 H, d), 7.23-7.28 ppm (2H, m), 6.90-6.99 ppm (2H, m), 4.44 ppm (2H, q), 1.43 ppm (3H, t).

## Patentansprüche

1. Verfahren zur Herstellung von 4-halogenalkyl-3-mercapto-substituierten 2-Hydroxybenzoesäure-Derivaten der allgemeinen Formel (I), **dadurch gekennzeichnet, dass** 4-thiosubstiuierte β-Ketoester der Formel (II) mit Alkoxyvinylhaloalkylketonen der Formel (III) bei einer Temperatur von ≥ -30°C in Gegenwart eines tertiären Amins und eines Lösungsmittels umgesetzt werden, und worin die Reste, Symbole und Indices wie nachfolgend definiert sind: 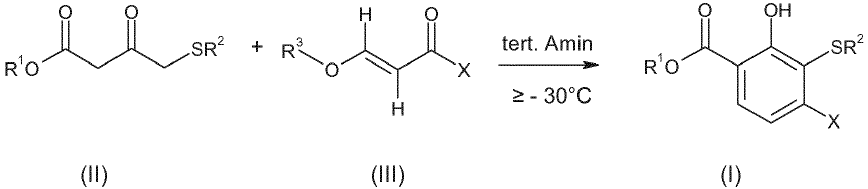
R¹, R² und R³ bedeuten unabhängig voneinander jeweils
durch p Reste aus der Gruppe bestehend aus Fluor, Chlor, (C₁-C₄)-Alkoxy und (C₃-C₇)-Cycloalkyl substituiertes (C₁-C₆)-Alkyl,
durch p Reste aus der Gruppe bestehend aus Fluor, Chlor, (C₁-C₄)-Alkyl, (C₁-C₄)-Alkoxy und (C₃-C₇)-Cycloalkyl substituiertes(C₃-C₇)-Cycloalkyl,
durch p Reste aus der Gruppe bestehend aus Fluor, Chlor, Brom, lod, (C₁-C₄)-Alkyl, (C₁-C₄)-Alkoxy, (C₃-C₇)-Cycloalkyl und (C₁-C₄)-Alkylthio substituiertes Phenyl, 1-Naphthyl, 2-Naphthyl oder 5- oder 6-gliedriges Heteroaryl, wobei dieses Heteroaryl ein oder zwei Heteroatome aus der Gruppe bestehend aus Sauerstoff und Stickstoff enthält,
X bedeutet Halogen-(C₁-C₄)-alkyl,
p bedeutet 0, 1, 2, 3 oder 4.

2. Verfahren nach Anspruch 1, worin die Reste, Symbole und Indices folgende Bedeutungen haben:
R¹, R² und R³ bedeuten unabhängig voneinander jeweils
durch p Reste aus der Gruppe bestehend aus Fluor, Chlor, (C₁-C₂)-Alkoxy und (C₃-C₇)-Cycloalkyl substituiertes (C₁-C₄)-Alkyl,
durch p Reste aus der Gruppe bestehend aus Fluor, Chlor, (C₁-C₂)-Alkyl und (C₁-C₂)-Alkoxy substituiertes(C₃-C₇)-Cycloalkyl,
durch p Reste aus der Gruppe bestehend aus Fluor, Chlor, Brom, lod, (C₁-C₂)-Alkyl, (C₁-C₂)-Alkoxy, (C₃-C₇)-Cycloalkyl und (C₁-C₂)-Alkylthio substituiertes Phenyl,
X bedeutet Fluor-(C₁-C₃)-alkyl oder Chlor-(C₁-C₃)-alkyl,
p bedeutet 0, 1, 2, 3 oder 4.

3. Verfahren nach Anspruch 1 oder 2, worin X für CF₃, CF₂H, CFH₂, CFCIH, CF₂CH₃, CF(CH₃)₂, CF₂CF₃ oder CH₂CF₃ steht.

4. Verfahren nach einem der Ansprüche 1 bis 3, wobei 0,1 bis 3 Äquivalente des tertiären Amins eingesetzt werden.

5. Verfahren nach einem der Ansprüche 1 bis 4, wobei 0,5 bis 1,5 Äquivalente des tertiären Amins eingesetzt werden.

6. Verfahren nach einem der Ansprüche 1 bis 5, wobei Acetonitril als Lösungsmittel eingestzt wird.

7. Verfahren nach einem der Ansprüche 1 bis 6, wobei die Verbindung der Formel (II) und das Amin in einem organischen Lösungsmittel vorgelegt werden und die Verbindung der Formel (III) unter Kühlung zugetropft wird.

8. Verfahren nach einem der Ansprüche 1 bis 7, wobei die Reaktion bei einer Temperatur von -30°C und 50°C durchgeführt wird.

9. Verfahren nach einem der Ansprüche 1 bis 8, wobei die Reaktion bei einer Temperatur von -10°C bis 30°C durchgeführt wird.

10. Verfahren nach einem der Ansprüche 1 bis 9, wobei die Reaktionsmischung nach Beendigung der Reaktion mit wässriger NaOH- oder KOH-Lauge behandelt wird.

11. Verbindungen der Formel (la) 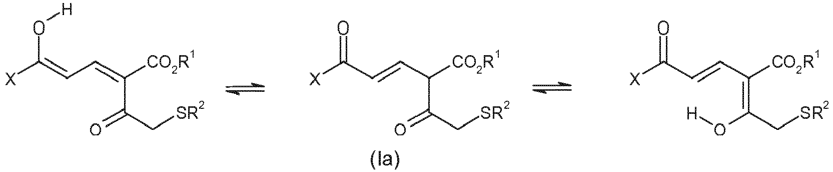 oder deren Tautomerer, worin die Substituenten R¹, R² und X wie in einem der Ansprüche 1 bis 3 definiert sind.

## Claims

1. Process for preparing 4-haloalkyl-3-mercapto-substituted 2-hydroxybenzoic acid derivatives of the general formula (I), **characterized in that** 4-thio-substituted β-keto esters of the formula (II) are reacted with alkoxyvinyl haloalkyl ketones of the formula (III) at a temperature of ≥ -30°C in the presence of a tertiary amine and of a solvent, and in which the radicals, symbols and indices are each defined as follows: 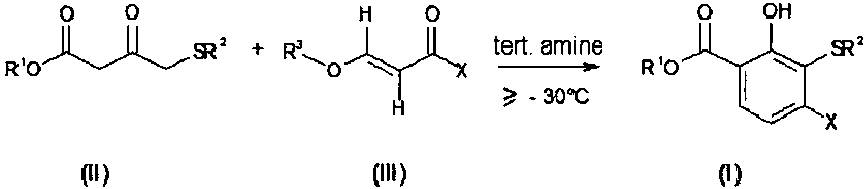
R¹, R² and R³ are each independently
(C₁-C₆)-alkyl substituted by p radicals from the group consisting of fluorine, chlorine, (C₁-C₄)-alkoxy and (C₃-C₇-cycloalkyl,
(C₃-C₇)-cycloalkyl substituted by p radicals from the group consisting of fluorine, chlorine, (C₁-C₄)-alkyl, (C₁-C₄) -alkoxy and (C3-C7 -cycloalkyl,
phenyl, 1-naphthyl, 2-naphthyl or 5- or 6-membered heteroaryl, each substituted by p radicals from the group consisting of fluorine, chlorine, bromine, iodine, (C₁-C₄) -alkyl, (C₁-C₄) -alkoxy, (C₃-C₇-cycloalkyl and (C₁-C₄)-alkylthio, where said heteroaryl contains one or two heteroatoms from the group consisting of oxygen and nitrogen,
X is halo- (C₁-C₄) -alkyl,
p is 0, 1, 2, 3 or 4.

2. Process according to Claim 1, in which the radicals, symbols and indices are each defined as follows:
R¹, R² and R³ are each independently
(C₁-C₄)-alkyl substituted by p radicals from the group consisting of fluorine, chlorine, (C₁-C₂)-alkoxy and
(C₃-C₇)-cycloalkyl,
(C₃-C₇)-cycloalkyl substituted by p radicals from the group consisting of fluorine, chlorine, (C₁-C₂)-alkyl and (C₁-C₂)-alkoxy,
phenyl substituted by p radicals from the group consisting of fluorine, chlorine, bromine, iodine, (C₁-C₂)-alkyl, (C₁-C₂)-alkoxy, (C₃-C₇)-cycloalkyl and
(C₁-C₂) -alkylthio,
X is fluoro- (C₁-C₃) -alkyl or chloro- (C₁-C₃) -alkyl,
p is 0, 1, 2, 3 or 4.

3. Process according to Claim 1 or 2, in which X is CF₃, CF₂H, CFH₂, CFC1H, CF₂CH₃, CF(CH₃)₂, CF₂CF₃ or CH₂CF₃.

4. Process according to any of Claims 1 to 3, wherein 0.1 to 3 equivalents of the tertiary amine are used.

5. Process according to any of Claims 1 to 4, wherein 0.5 to 1.5 equivalents of the tertiary amine are used.

6. Process according to any of Claims 1 to 5, wherein acetonitrile is used as the solvent.

7. Process according to any of Claims 1 to 6, wherein the compound of the formula (II) and the amine are initially charged in an organic solvent and the compound of the formula (III) is added dropwise while cooling.

8. Process according to any of Claims 1 to 7, wherein the reaction is performed at a temperature of -30°C and 50°C.

9. Process according to any of Claims 1 to 8, wherein the reaction is performed at a temperature of -10°C to 30°C.

10. Process according to any of Claims 1 to 9, wherein the reaction mixture is treated after the reaction has ended with aqueous NaOH solution or KOH solution.

11. Compounds of the formula (Ia) 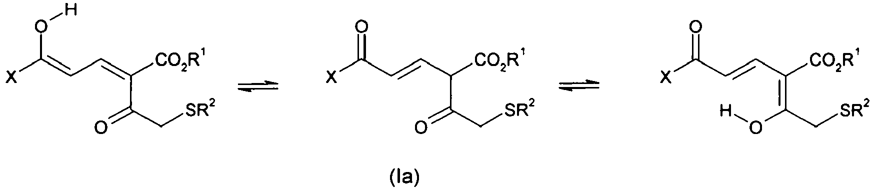 or tautomers thereof, in which the substituents R¹, R² and X are each as defined in any of Claims 1 to 3.

## Revendications

1. Procédé de fabrication de dérivés de l'acide 2-hydroxybenzoïque à substitution 4-halogénoalkyl-3-mercapto de formule générale (I), **caractérisé en ce que** des β-cétoesters à substitution 4-thio de formule (II) sont mis en réaction avec des alcoxyvinylhaloalkylcétones de formule (III) à une température ≥ -30 °C en présence d'une amine tertiaire et d'un solvant, et dans lequel les radicaux, les symboles et les indices sont définis de la manière suivante : 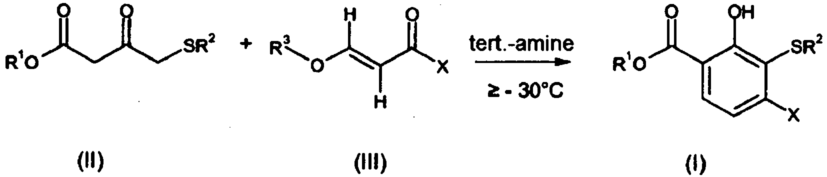
R¹, R² et R³ signifient chacun indépendamment les uns des autres
alkyle en (C₁-C₆) substitué par p radicaux du groupe constitué par fluor, chlore, alcoxy en (C₁-C₄) et cycloalkyle en (C₃-C₇),
cycloalkyle en (C₃-C₇) substitué par p radicaux du groupe constitué par fluor, chlore, alkyle en (C₁-C₄), alcoxy en (C₁-C₄) et cycloalkyle en (C₃-C₇), phényle, 1-naphtyle, 2-naphtyle ou hétéroaryle à 5 ou 6 éléments substitué par p radicaux du groupe constitué par fluor, chlore, brome, iode, alkyle en (C₁-C₄), alcoxy en (C₁-C₄), cycloalkyle en (C₃-C₇) et alkylthio en (C₁-C₄), cet hétéroaryle contenant un ou deux hétéroatomes du groupe constitué par l'oxygène et l'azote,
X signifie halogéno-alkyle en (C₁-C₄),
p signifie 0, 1, 2, 3 ou 4.

2. Procédé selon la revendication 1, dans lequel les radicaux, les symboles et les indices ont les significations suivantes :
R¹, R² et R³ signifient chacun indépendamment les uns des autres
alkyle en (C₁-C₄) substitué par p radicaux du groupe constitué par fluor, chlore, alcoxy en (C₁-C₂) et cycloalkyle en (C₃-C₇),
cycloalkyle en (C₃-C₇) substitué par p radicaux du groupe constitué par fluor, chlore, alkyle en (C₁-C₂) et alcoxy en (C₁-C₂),
phényle substitué par p radicaux du groupe constitué par fluor, chlore, brome, iode, alkyle en (C₁-C₂),
alcoxy en (C₁-C₂), cycloalkyle en (C₃-C₇) et alkylthio en (C₁-C₂),
X signifie fluoro-alkyle en (C₁-C₃) ou chloro-alkyle en (C₁-C₃),
p signifie 0, 1, 2, 3 ou 4.

3. Procédé selon la revendication 1 ou 2, dans lequel X représente CF₃, CF₂H, CFH₂, CFC1H, CF₂CH₃, CF (CH₃)₂, CF₂CF₃ ou CH₂CF₃.

4. Procédé selon l'une quelconque des revendications 1 à 3, dans lequel 0,1 à 3 équivalents de l'amine tertiaire sont utilisés.

5. Procédé selon l'une quelconque des revendications 1 à 4, dans lequel 0,5 à 1,5 équivalents de l'amine tertiaire sont utilisés.

6. Procédé selon l'une quelconque des revendications 1 à 5, dans lequel l'acétonitrile est utilisé en tant que solvant.

7. Procédé selon l'une quelconque des revendications 1 à 6, dans lequel le composé de formule (II) et l'amine sont chargés dans un solvant organique et le composé de formule (III) est ajouté goutte à goutte sous refroidissement.

8. Procédé selon l'une quelconque des revendications 1 à 7, dans lequel la réaction est réalisée à une température de -30 °C et 50 °C.

9. Procédé selon l'une quelconque des revendications 1 à 8, dans lequel la réaction est réalisée à une température de -10 °C à 30 °C.

10. Procédé selon l'une quelconque des revendications 1 à 9, dans lequel le mélange réactionnel est traité avec une lessive de NaOH ou de KOH aqueuse après la fin de la réaction.

11. Composés de formule (Ia) 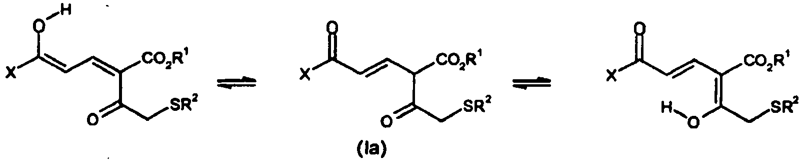 ou leurs tautomères, les substituants R¹, R² et X étant tels que définis dans l'une quelconque des revendications 1 à 3.
